(19) Europäisches Patentamt
European Patent Office
Office européen des brevets

(11) EP 4 233 917 A1

# (12) EUROPEAN PATENT APPLICATION

(43) Date of publication:
**30.08.2023 Bulletin 2023/35**

(21) Application number: **22382150.5**

(22) Date of filing: **23.02.2022**

(51) International Patent Classification (IPC):
***A61K 47/64*** *(2017.01)* ***A61P 31/14*** *(2006.01)*

(52) Cooperative Patent Classification (CPC):
**A61K 47/645; A61P 31/14**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **Universitat Pompeu Fabra**
  **08002 Barcelona (ES)**
- **Instituto de Medicina Molecular João Lobo Antunes**
  **1649-028 Lisboa (PT)**
- **Instituto de Bioquimica Médica Leopoldo de Meis**
  **21941-902 Rio de Janeiro (BR)**
- **Faculdade de Medicina da Universidade de Lisboa**
  **1649-028 Lisboa (PT)**

(72) Inventors:
- **ANDREU MARTÍNEZ, David**
  **E-08002 Barcelona (ES)**
- **TODOROVSKI, Toni**
  **E-08002 Barcelona (ES)**
- **CRUZ OLIVEIRA, Christine**
  **1649-028 Lisboa (PT)**
- **THOMPSON DA POIAN, Andrea**
  **21941-902 Rio de Janeiro (BR)**
- **DE OLIVEIRA FERNANDES SIQUEIRA, Lorena**
  **21941-902 Rio de Janeiro (BR)**
- **RICO BOTAS CASTANHO, Miguel Augusto**
  **1649-028 Lisboa (PT)**
- **DE MENDONÇA, Diogo Alexandre**
  **1649-028 Lisboa (PT)**

(74) Representative: **ABG Intellectual Property Law, S.L.**
**Avenida de Burgos, 16D**
**Edificio Euromor**
**28036 Madrid (ES)**

Remarks:
The complete document including Reference Table(s) and the Sequence Listing(s) can be downloaded from the EPO website

# (54) ANTI-INFECTIVE CONJUGATE FOR USE IN THE TREATMENT OF ZIKA VIRUS INFECTION

(57) The present invention relates to a conjugate of protoporphyrin IX with a peptide or a salt or a co-crystal thereof for use in the prevention or treatment of Zika virus infection. It also relates to a pharmaceutical composition comprising said conjugate and a pharmaceutically acceptable diluent or carrier for use in the prevention or treatment of Zika virus infection.

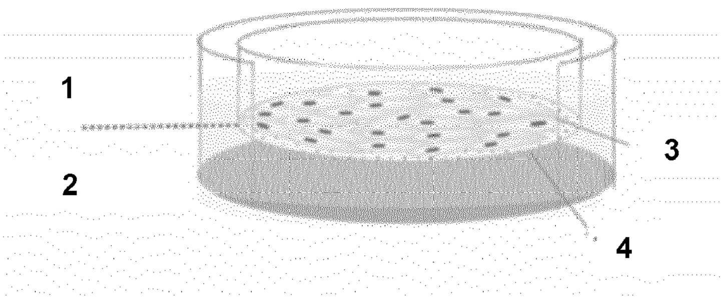


FIG. 1

EP 4 233 917 A1

## Description

## FIELD OF THE INVENTION

The present invention relates to a conjugate of protoporphyrin IX with a peptide or a pharmaceutically acceptable salt or a pharmaceutically acceptable co-crystal thereof for use in the prevention or treatment of Zika virus infection. It also relates to a pharmaceutical composition comprising said conjugate and a pharmaceutically acceptable diluent or carrier for use in the prevention or treatment of Zika virus infection.

## BACKGROUND

Zika virus (ZIKV) infection is caused by an enveloped virus of the *Flaviviridae* family transmitted by *Aedes* mosquito vectors and is associated with eponymous fever. Direct consequences of infection by Zika Virus are of particular concern in pregnant women, for infection may cause foetal loss, pre-term birth and irreversible foetal brain abnormalities, such as microcephaly. Infection may also provoke the development of the Guillain-Barre syndrome or encephalomyelitis in patients. Because of its mode of propagation, the Zika virus infection holds great epidemic potential. While certain inactive and/or "non-live" vaccines are being developed to prevent the spread of ZIKV, there is to date no available therapy for the treatment of infection by ZIKV and its related diseases. There is consequently a strong medical need for the provision of anti-infective agents able to prevent the development of the Zika virus infection in patients, and in particular in pregnant women.

It is known in the art that the treatment of the Zika virus related disease requires the potential therapy to have the ability to cross the blood-brain barrier (BBB) so that the therapy reaches the virus at its most active location. In the case of pregnant women and foetal infection, said therapy shall in addition have the capacity to cross the blood-placental barrier (BPB). The BBB is constituted by endothelial cells in charge of protecting the brain from toxins, pathogens, inflammation, injury and disease, while regulating the homeostasis of the central nervous system. The blood brain barrier contains highly selective carriers for a limited number of molecular entities found in the blood, thereby frequently preventing active therapeutic agents from acceding to the brain. The blood-placental barrier is known to prevent large molecules from entering the placenta.

Bioactive compounds suitable for treating Zika virus have been reported in the art. In particular, Neris and co-workers have reported that protoporphyrin IX metallated with cobalt (CoPPIX) or tin (SnPPIX), are efficient inactivators of Zika virus by targeting the envelope of the virus. Treatment of ZIKV with CoPPIX or SnPPIX results in the loss of viral envelope protein, affects the morphology of the virus, and also blocks penetration into host cells. Without light activation, CoPPIX and SnPPIX exhibit an $IC_{50}$ value for inactivation of ZIKV of 2.49 and 5.98 $\mu$M respectively. With light activation, CoPPIX and SnPPIX exhibit an $IC_{50}$ value for inactivation of ZIKV of 5.79 and 0.16 $\mu$M respectively. The authors are however silent about the ability of these compounds to cross the BBB and/or the BPB and thus provide no evidence that the compounds could be useful in the treatment of viral infections (in-vivo). In addition, the authors do neither disclose nor discuss the biological activity against ZIKV of the non-metallated protoporphyin IX.

In this regard, non-metallated protoporphyrin IX has been disclosed in the art by Cruz-Oliveira and co-workers as inactivator of Vesicular Stomatitis Virus, exhibiting an $IC_{50}$ of 8.294 nM without light irradiation and 0.09764 nM under light irradiation. The disclosed mechanism of action includes porphyrin intercalation into the virus envelope lipid bilayer with changes in lipid ordering and singlet oxygen production after their photoactivation. The paper is also silent on the ability of the products to cross the BBB and, as a consequence, of their ability to effectively treat viral infections.

In a different approach, peptidic inactivators of ZIKV have been developed. Yu and co-workers disclosed in 2017 a peptide Z2 derived from the stem region of the E protein of ZIKV. The stem region of E protein is known in the art for its ability to anchor to and fuse with the membrane of ZIKV. Z2 has the following sequence: MAVLGDTAWDFGSVGGALNSLGKGIHQIFGAAF. This sequence is reported as suitable for inactivating ZIKV, having an $IC_{50}$ of between 1.75 $\mu$M and 13.91 $\mu$M for different strains of ZIKV, while avoiding vertical transmission of the virus to the foetus in pregnant mice. Z2 is disclosed as having the potential to inactivate ZIKV before or after the virions have penetrated the placenta.

Conjugates of cell- penetrating peptides with antiviral agents for use in the treatment of ZIKV related disease have been reported by Schroeder and co-workers. The disclosed conjugates combine the antiviral activity of peptidomimetic inhibitors and cell-penetrating peptides. In this approach, dextran is used as an anchoring point for the conjugates, which are provided in the form of nanoparticles. The authors are however silent about the ability of the provided conjugates to cross the blood-brain barrier or the blood-placenta barrier.

From what is disclosed in the art, it derives that there is still a need for providing improved conjugates and pharmaceutical compositions therefrom for use in the treatment or prevention of Zika virus infection.

SUMMARY OF THE INVENTION

After exhaustive research, the inventors have developed a porphyrin-peptide conjugate exhibiting inhibition activity against Zika virus infection. Advantageously, the developed compound is able to cross both blood-brain and blood-placental barriers (BBB and BPB) and is stable in serum for a period of at least six hours. In particular, it exhibits a half-life time of about 22h. Said compound is therefore suitable for preventing and treating foetal infection by ZIKV in pregnant women.

The conjugate compound of the invention comprises in its molecular structure a non-metallated protoporphyrin IX unit and a peptide shuttle for penetrating the blood-brain barrier and the blood-placental barrier. As further discussed below in the Comparative Examples, non-conjugated protoporphyrin IX is poorly active in inhibiting ZIKV infection. However, the inventors have found that, when protoporhyrin IX is conjugated to a peptide shuttle suitable for penetrating the blood-brain barrier and having as sequence Val-Gln-Gln-Leu-Thr-Lys-Arg-Phe-Ser-Leu-$NH_2$ (SEQ ID NO1-$NH_2$), the resulting conjugate is not only suitable for crossing the blood-brain barrier, but also the activity of the conjugate in inhibiting infection by Zika virus is increased significantly in an unexpected and surprising manner. It is unexpected because the non-metallated and unconjugated protoporphyrin IX shows poor activity in inhibiting the infection by Zika virus infection. It is thus plausible that the conjugation with the peptide having as sequence SEQ ID NO1-$NH_2$, which is suitable for penetrating both the blood-brain barrier and the blood-placental barrier, is essential to the inhibiting activity of the provided conjugate. The inventors therefore believe that the role of the peptide in the conjugate compound of the invention goes beyond the mere contribution of carrying the protoporphyrin IX through the blood-brain barrier and the blood-placental barrier, and thereby contributes to the activity of the conjugate compound of the invention in inhibiting ZIKV infection.

Thus, in a first aspect, the invention relates to a conjugate of protoporphyrin IX with the peptide having the sequence Val-Gln-Gln-Leu-Thr-Lys-Arg-Phe-Ser-Leu-$NH_2$ (SEQ ID NO1-$NH_2$) or a pharmaceutically acceptable salt or a pharmaceutically acceptable co-crystal thereof for use in the prevention or treatment of Zika virus infection.

The invention also relates in a second aspect to a composition comprising a conjugate of protoporphyrin IX with the peptide having the sequence Val-Gln-Gln-Leu-Thr-Lys-Arg-Phe-Ser-Leu-$NH_2$ (SEQ ID NO1-$NH_2$) or a pharmaceutically acceptable salt or a pharmaceutically acceptable co-crystal thereof and a pharmaceutically acceptable diluent or carrier for use in the prevention or treatment of Zika virus infection.

In a third aspect, the invention relates to the use of the conjugate as defined in any particular and preferred embodiment of the first aspect of the invention for the manufacture of a medicament or a pharmaceutical composition as defined in any particular and preferred embodiment of the second aspect of the invention for treating or preventing infection by Zika virus.

In a fourth aspect, the invention relates to a method for treating and/or preventing infection by Zika virus which comprises administrating the conjugate compound as defined in any particular and preferred embodiment of the first aspect of the invention or a pharmaceutical composition as defined in any particular and preferred embodiment of the second aspect of the invention.

**BRIEF DESCRIPTION OF THE DRAWINGS**

Fig. 1 describes the experimental set-up for measurement of BBB and BPB translocation of the conjugates whereby a monolayer of cells (3) in a transwell insert (4) separates the basolateral side (bottom, 2) from the apical side (top, 1), and whereby the amount of conjugate crossing the cellular monolayer from the apical side to the basolateral side is measured.

**DETAILED DESCRIPTION**

All terms as used herein in this application, unless otherwise stated, shall be understood in their ordinary meaning as known in the art. Other more specific definitions for certain terms as used in the present application are as set forth below and are intended to apply uniformly throughout the specification and claims unless an otherwise expressly set out definition provides a broader definition.

For the purposes of the invention, any ranges given include both the lower and the upper end-points of the range. Ranges given, such as temperatures, times, molar ratio, volume ratio and the like, should be considered approximate (i.e. with a 5% margin of variation around indicated point), unless specifically stated.

In this description, the abbreviations used for amino acids follow the recommendations of the 1983 IUPAC-IUB Commission of Biochemical Nomenclature specified in Eur. J. Biochem., (1984), 138, 937.

Thus, for example, Arg or R represents $NH_2$-CH($CH_2$-$CH_2$-$CH_2$-NH-C(=NH)$NH_2$)-COOH, Arg- or R- represents $NH_2$-CH($CH_2$-$CH_2$-$CH_2$-NH-C(=NH)$NH_2$)-CO-, -Arg or -R represents -NH-CH($CH_2$-$CH_2$-$CH_2$-NH-C(=NH)$NH_2$)-COOH, and -Arg- or -R- represents -NH-CH($CH_2$-$CH_2$-$CH_2$-NH-C(=NH)$NH_2$)-CO-. Therefore, the hyphen, which represents the

peptide bond, eliminates the OH in the 1-carboxyl group of the amino acid (represented here in the conventional non-ionized form) when situated to the right of the symbol, and eliminates the H of the 2-amino group of the amino acid when situated to the left of the symbol; both modifications can be applied to the same symbol (see Table 1).

The amino acids are named using the conventional nomenclature in one and/or three letter codes, as follows:

- alanine, Ala or A,
- arginine, Arg or R.
- asparagine, Asn or N,
- aspartic acid, Asp or D,
- glutamine, Gln or Q,
- glycine, Gly or G,
- histidine, His or H,
- isoleucine, Ile or I
- leucine, Leu or L,
- lysine, Lys or K,
- methionine, Met or M,
- phenylalanine, Phe or F,
- proline, Pro or P,
- serine, Ser or S
- threonine, Thr or T
- tyrosine, Tyr or Y
- tryptophan, Trp or W,
- valine, Val or V.

In the context of the present invention, the term ($C_1$-$C_{12}$)alkyl refers to a saturated, linear or branched, hydrocarbon chain having from 1 to 12 carbon atoms. Non-limiting examples of ($C_1$-$C_{12}$)alkyl groups include methyl, ethyl, propyl, iso -propyl, n-butyl, tert -butyl, iso -butyl, sec -butyl, n -pentyl, tert -pentyl, iso -pentyl, sec -pentyl, neo pentyl, 1-ethylpropyl, n -hexyl, 1-methyl-pentyl, 2-methyl-pentyl, 3-methyl-pentyl, 4-methyl-pentyl, 3,3-dimethylbutyl, 2,2-dimethylbutyl, 1,1-dimethylbutyl, 2,3-dimethylbutyl, 1,3-dimethylbutyl, 1,2-dimethylbutyl, 3-ethylbutyl, 2-ethylbutyl, 1-ethylbutyl, n -heptyl, n -octyl, n -nonyl, n-decanyl, undecanyl, and dodecanyl.

In the context of the present invention, the term ($C_2$-$C_{12}$)alkenyl refers to a saturated, linear or branched, hydrocarbon chain having from 2 to 12 carbon atoms and further comprising a carbon-carbon double bond.

The term "cycloalkyl" refers to a monocyclic or saturated bicyclic cycloalkyl group having the number of carbon atoms mentioned in the description. Examples of cycloalkyl groups include cyclopropyl, cyclobutyl, cyclopentyl, cyclohexyl, cycloheptyl and cyclooctyl.

As defined above, a first aspect of the invention relates to a conjugate of protoporphyrin IX with the peptide having the sequence Val-Gln-Gln-Leu-Thr-Lys-Arg-Phe-Ser-Leu-$NH_2$ (SEQ ID NO1-$NH_2$) or a pharmaceutically acceptable salt or a pharmaceutically acceptable co-crystal thereof for use in the prevention or treatment of ZIKA virus infection.

In a particular embodiment of the first aspect of the invention, the conjugate is one wherein protoporphyrin IX is linked to one conjugation site selected from the group consisting of the *N*-terminal valine of the peptide having SEQ ID NO1-$NH_2$, the side chains of the threonine or serine amino acids comprised in the peptide having SEQ ID NO1-$NH_2$, and the side chain of the lysine amino acid comprised in the peptide having SEQ ID NO1-$NH_2$.

When the conjugation site is the *N*-terminal valine of the peptide having SEQ ID NO1-$NH_2$ or the side chain of the lysine amino acid comprised in the peptide having SEQ ID NO1-$NH_2$, said conjugation site comprises an amino group, and said conjugation may take place via an amide functional group or a sulphonamide functional group. In such embodiments, it is preferred that the conjugation takes place via an amide group.

When the conjugation site is one of the side chains of the threonine or serine amino acids comprised in the peptide having SEQ ID NO1-$NH_2$, said conjugation site comprises a hydroxyl group, and said conjugation may take place via an ester or a sulfonate functional group. In such embodiments, it is preferred that the conjugation takes place via an ester group.

In a particular embodiment of the first aspect of the invention, the conjugate is one wherein protoporphyrin IX is linked to the *N*-terminal valine of the peptide having SEQ ID NO1-$NH_2$. Preferably, the conjugate is one wherein protoporphyrin IX is linked to the *N*-terminal valine of the peptide having SEQ ID NO1-$NH_2$ via an amide group.

In other particular embodiments of the first aspect of the invention, the conjugate is one wherein said protoporphyrin IX is linked to the peptide through one of its carboxylic acid groups.

In a more particular embodiment of the first aspect of the invention, the conjugate is one wherein protoporphyrin IX is linked to the *N*-terminal valine of the peptide having SEQ ID NO1-$NH_2$ through one of its carboxylic acid groups.

In other particular embodiments of the first aspect of the invention, the conjugate is a compound of formula A

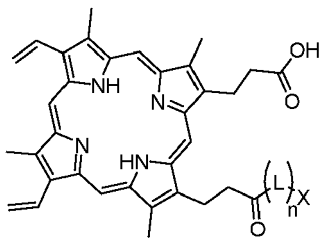

(A)

wherein n is 0 or 1;
X is selected from the group consisting of the radicals of formulae $X_1$, $X_2$, $X_3$ and $X_4$

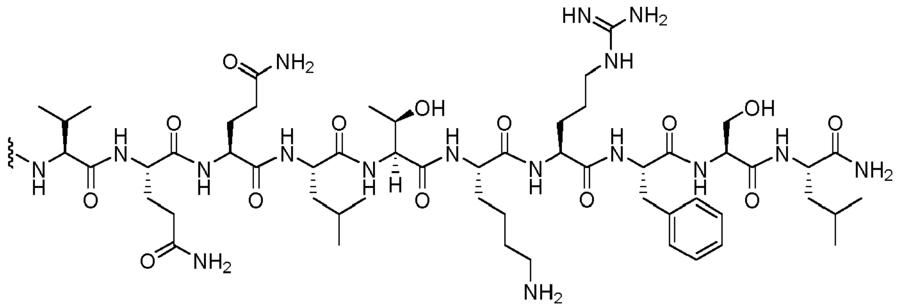

$X_1$

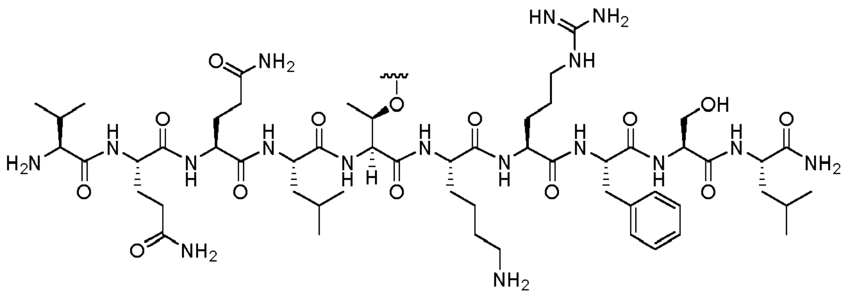

$X_2$

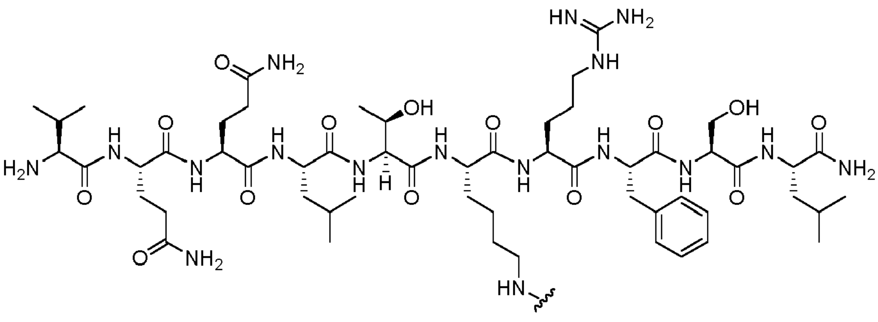

$X_3$

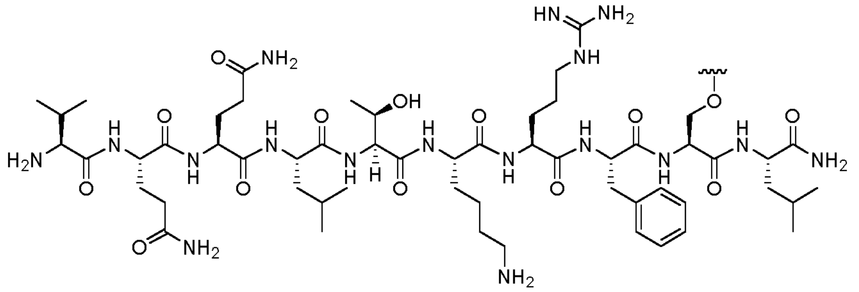

$X_4$

wherein each wavy line represents the attachment point to the remainder of the compound of formula A;
and wherein L represents a diradical of formula $-Y_1-L_1-CO-$ or $-Y_1-L_1-SO_2-$, wherein $Y_1$ is O or NH and is attached to the carbonyl group born by the protoporphyrin IX fragment in the compound of formula A and wherein the CO or $SO_2$ group is attached to the radical of formula X in the compound of formula A and wherein $L_1$ is a diradical of formula $-(CH_2-)_l(CH_2-CH_2-O-)_m(CH_2)_{l'}-$ where l and l' are each independently select from the integers of 0 to 3 and m is an integer of from 1 to 6; or, alternatively, $L_1$ is a diradical deriving from a radical selected from the group consisting $(C_1-C_{12})$alkyl, $(C_3-C_8)$cycloalkyl and $(C_2-C_{12})$alkenyl.

In other particular embodiments of the first aspect of the invention, the conjugate is a compound of formula A as defined above wherein n is 0.

In other particular embodiments of the first aspect of the invention, the conjugate is a compound of formula A as defined above wherein X is $X_1$ or $X_3$. Preferably, the conjugate is a compound of formula A as defined above wherein X is $X_1$. In these embodiments, conjugation is made via an amide bond, which is advantageously more resistant to enzymatic cleavage than ester bonds. In addition, when X is $X_1$ in the compound of formula A, it has been observed that the formed conjugate is particularly stable in serum.

In other particular embodiments of the first aspect of the invention, the conjugate is a compound of formula A as defined above wherein n is 0 and X is $X_1$. In such embodiments, the conjugate is a compound of formula (I)

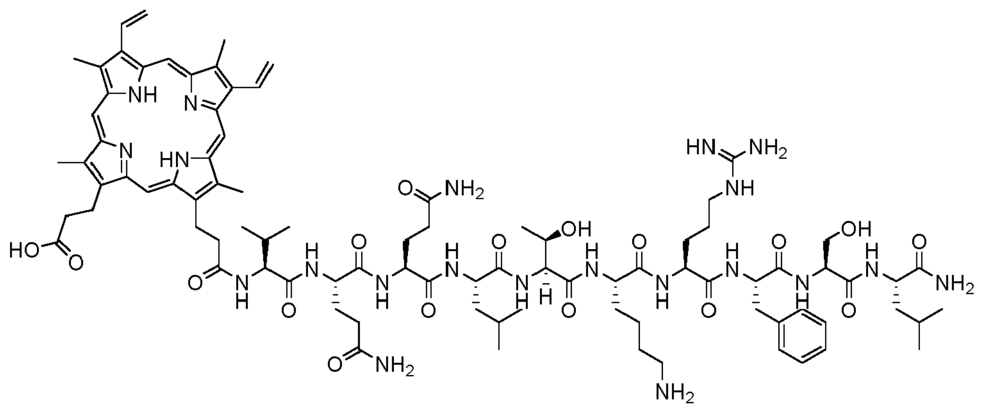

(I)

In further particular embodiments of the first aspect of the invention, the conjugate is a compound of formula A as defined above wherein n is 1.

In further particular embodiments of the first aspect of the invention, the conjugate is a compound of formula A as defined above wherein L is a diradical of formula $-Y_1-L_1-CO-$.

In further particular embodiments of the first aspect of the invention, the conjugate is a compound of formula A as defined above wherein $Y_1$ is NH.

In further particular embodiments of the first aspect of the invention, the conjugate is a compound of formula A as defined above wherein $L_1$ is a diradical of formula $-(CH_2-)_l(CH_2-CH_2-O-)_m(CH_2)_{l'}-$ where l and l' are each independently select from the integers of 0 to 3 and m is an integer of from 1 to 6. Preferably, l is 0. Also preferably l' is 1. Also preferably, m is 2.

In further particular embodiments of the first aspect of the invention, the conjugate is a compound of formula A as defined above wherein n is 1, L is a diradical of formula $-Y_1-L_1-CO-$ wherein $Y_1$ is NH.

In further particular embodiments of the first aspect of the invention, the conjugate is a compound of formula A as defined above wherein n is 1, L is a diradical of formula $-Y_1-L_1-CO-$ wherein $Y_1$ is NH and $L_1$ is a diradical of formula

$-(CH_2-)_l(CH_2-CH_2-O-)_m(CH_2)_{l'}-$ where l and l' are each independently select from the integers of 0 to 3 and m is an integer of from 1 to 6.

In further particular embodiments of the first aspect of the invention, the conjugate is a compound of formula A as defined above wherein n is 1, L is a diradical of formula $-Y_1-L_1-CO-$ wherein $Y_1$ is NH and $L_1$ is a diradical of formula $-(CH_2-)_l(CH_2-CH_2-O-)_m(CH_2)_{l'}-$ where l is 0 and l' is 1.

In further particular embodiments of the first aspect of the invention, the conjugate is a compound of formula A as defined above wherein n is 1, L is a diradical of formula $-Y_1-L_1-CO-$ wherein $Y_1$ is NH and $L_1$ is a diradical of formula $-(CH_2-)_l(CH_2-CH_2-O-)_m(CH_2)_{l'}-$ where l is 0, l' is 1 and m is 2.

A method for the preparation of a conjugate according to the first aspect of the invention is also provided. Said method comprises the following steps:

(i) providing a peptide having the sequence VQQLTKRFSL-$NH_2$ (SEQ ID NO1-$NH_2$);
(ii) providing protoporphyrin IX;
(iii) optionally, coupling the protoporhyrin provided in step (ii) with a linker compound suitable for being coupled to protoporphyrin IX and the peptide of sequence SEQ ID NO1-$NH_{2;}$ and
(iv) reacting the peptide provided in step (i) with the compound provided in step (ii) or in step (iii) to produce said conjugate.

Alternatively, and also preferably, the compound of the invention may be prepared by a method comprising the following steps:

(i) providing a peptide having the sequence VQQLTKRFSL-$NH_2$ (SEQ ID NO1-$NH_2$);
(ii) providing protoporphyrin IX;
(iii) optionally, coupling the peptide provided in step (i) with a linker compound suitable for being coupled to protoporphyrin IX and the peptide of sequence SEQ ID NO1-$NH_{2;}$ and
(iv) reacting protoporphyrin IX provided in step (ii) with the compound provided in step (ii) or in step (iii) to produce said conjugate.

The peptide of sequence SEQ ID NO1-$NH_2$ may be prepared by solid phase synthesis following well-known procedures. As will become apparent to the skilled person, adequate protection of side chain residues may be necessary during the performance of steps (i), (iii) and (iv), in particular for the side chains of the amino acids forming the peptide bearing hydroxyl, amino, guanidine or amide groups. Thus, said method may further include several protection and deprotection steps as well as the use of amino acids bearing protecting groups as reagents. In particular, the trityl (Trt) group is suitable for protecting amide functional groups comprised in the side chains of Gln. Also particularly, the *tert*-butyl group is suitable for protecting the hydroxyl groups comprised in the side chains of Ser and Thr. Also particularly, the *tert*-butylcarboxy (Boc) group is suitable for protecting the amine groups comprised in the side chains of Lys. Also particularly, the 2,2,4,6,7-pentamethyl-2,3-dihydrobenzofuran-5-sulfonyl group (Pbf) is suitable for protecting the guanidine groups comprised in the side chains of Arg. Procedures for introducing and removing the aforementioned groups are well known in the art and will become apparent to the skilled person.

In optional step (iii), protoporphyrin IX may be coupled with a linker compound via the formation of an ester, i.e. by reaction of an acyl chloride deriving from protoporphyrin IX with a hydroxyl group comprised in the linker compound. Protoporphyrin IX may also be coupled with a linker compound via the formation of an amide, i.e. by reaction of an acyl chloride deriving from protoporphyrin IX with an amine group comprised in the linker compound or, alternatively, by reaction of protoporphyrin IX with an amine group comprised in the linker compound in the presence of reagents for peptidic coupling. Protoporphyin IX may be coupled with a linker compound via the formation of an alkene, i.e. by metathesis reaction of protoporphyrin IX with an alkene group comprised in the linker compound. In particular, the linker compound of step (iii) may be a compound of formula $HY_1-L_1-COOH$ or $HY_1-L_1-SO_3H$ wherein $Y_1$ and $L_1$ are as defined above in the first aspect of the invention.

Similarly, in an alternative optional step (iii), the peptide of sequence SEQ ID NO1-$NH_2$ may be coupled with a linker compound via the formation of an ester through an hydroxyl group comprised in a side chain by reaction with an acyl chloride group comprised in the linker compound. The peptide of sequence SEQ ID NO1-$NH_2$ may be coupled with a linker compound via the formation of an amide, i.e. by reaction of an acyl chloride comprised in the linker compound with an amine group comprised in the peptide of SEQ ID NO1-$NH_2$ or, alternatively, by reaction of a carboxylic acid group comprised in the linker compound with an amine group comprised in the peptide of SEQ ID NO1-$NH_2$ in the presence of reagents for peptidic coupling.

Step (iv) may be carried out by peptidic coupling by contacting the peptide provided in step (i) with the compound provided in step (ii) or in step (iii) in the presence of reagents for peptidic coupling. This is particularly the case when the conjugation site in the peptide of sequence SEQ ID NO1-$NH_2$ is an amino group born either by the *N*-terminal valine

or by the side chain of the lysine amino acid.

Alternatively, when the conjugation site in the peptide of sequence SEQ ID NO1-$NH_2$ is a hydroxyl group borne by the Ser or Thr amino acid side chain, step (iv) may be carried out by contacting one of the acyl or sulfonyl chlorides of formula PP'Cl, PP'-$Y_1$-COCl and PP'-$Y_1$-$SO_2$Cl (wherein PP' stands for the acyl radical resulting from omitting the OH of one of the carboxylic acid groups of protoporphyrin IX) with the peptide.

Step (iv) of the preparation of the conjugate of the invention when X is $X_1$ in the compound of formula A may thus follow the following scheme:

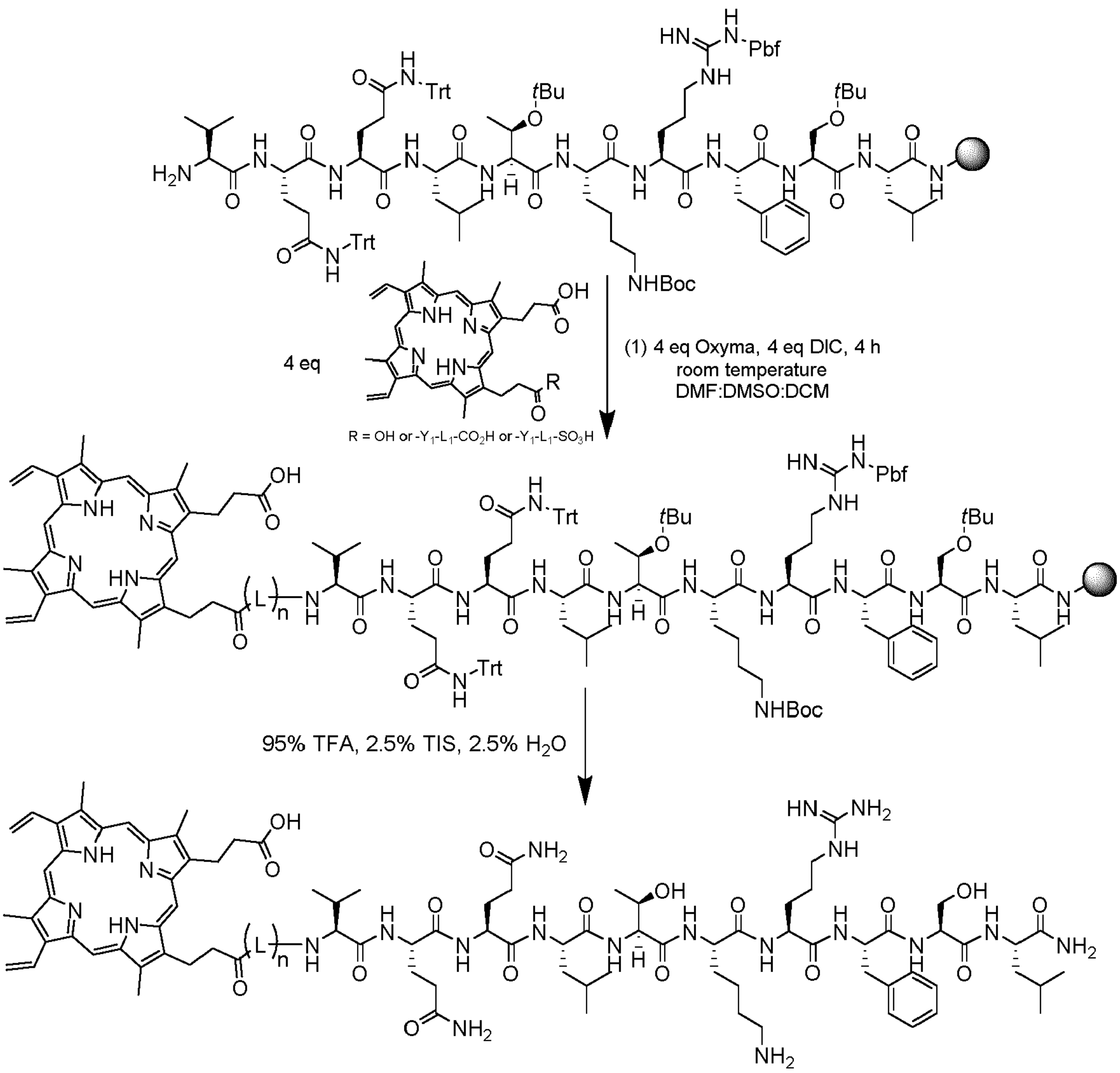


As will be apparent to the skilled person, a similar procedure may be followed when preparing the compound of formula A wherein X is $X_3$ starting from the same peptide of sequence SEQ ID NO1-$NH_2$ except that the amine group of Val is protected and the amine group of Lys is unprotected.

Similarly, a similar procedure may be followed when preparing the compound of formula A wherein X is $X_2$ or $X_4$ starting from the same peptide of sequence SEQ ID NO1-$NH_2$ except that the amine group of Val is protected and the hydroxyl group of Thr (when X is $X_2$) or Ser (when X is $X_4$) is unprotected and that the protoporphyrin IX reagent used in step (iv) is one wherein R is selected from Cl, -$Y_1$-$L_1$-COCl and -$Y_1$-$L_1$-$SO_2$Cl, where $Y_1$ and $L_1$ are as defined above.

As further detailed below, the conjugate compound of the invention is useful as anti-infective agent for Zika virus.

As detailed above, the second aspect of the invention thus relates to a composition comprising a conjugate of protoporphyrin IX with the peptide having the sequence Val-Gln-Gln-Leu-Thr-Lys-Arg-Phe-Ser-Leu-$NH_2$ (SEQ ID NO1-$NH_2$) or a pharmaceutically acceptable salt or a pharmaceutically acceptable co-crystal thereof and a pharmaceutically acceptable diluent or carrier for use in the prevention or treatment of Zika virus infection.

The composition according to the present invention may be in any form suitable for the application to humans

and/or animals, preferably humans including infants, children and adults, more particularly, pregnant women and can be produced by standard procedures known to those skilled in the art. Therefore, the composition in accordance with the invention may be adapted for topical or systemic application, particularly for dermal, transdermal, subcutaneous, intramuscular, intra-articular, intraperitoneal, intravenous, intra-arterial, intravesical, intraosseous, intracavernosal, pulmonary, buccal, sublingual, ocular, intravitreal, intranasal, percutaneous, rectal, vaginal, oral, epidural, intrathecal, intraventricular, intracerebral, intracerebroventricular, intracisternal, intraspinal, perispinal, intracranial, delivery via needles or catheters with or without pump devices, or other application routes.

The composition according to the second aspect of the invention may further be transferred into usual formulations, such as tablets, sugar-coated tablets, pills, granules, aerosols, syrups, emulsions, suspensions, solutions, ointments, creams, dermal patches and gel of any kind, using in particular essentially non-toxic and pharmaceutically acceptable carriers and diluents.

The composition of the second aspect of the invention may in certain embodiments further comprise auxiliary materials or additives of a pharmaceutical composition selected among excipients, support materials, lubricants, fillers, solvents, colorants, flavour conditioners such as sugars, antioxidants, binders, adhesives, disintegrants, anti-adherents, glidants and/or agglutinants. In the case of suppositories, this may imply waxes or fatty acid esters or preservatives, emulsifiers and/or carriers for parenteral application. The selection of these auxiliary materials and/or additives and the amounts to be used will depend on the form of application of the pharmaceutical composition and will become apparent to the skilled person.

In an embodiment the pharmaceutical compositions of the second aspect of the invention are a form suitable for oral administration, either solid or liquid. Suitable dose forms for oral administration may be tablets, pills, caplets, gel caps, chewing gums, capsules, granules, drops, syrups or solutions and may contain conventional excipients known in the art such as binding agents, for example syrup, acacia, gelatin, sorbitol, tragacanth, or polyvinylpyrrolidone; fillers, for example lactose, sugar, maize starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulfate.

The solid oral compositions may be prepared by conventional methods of blending, filling or tabletting. Repeated blending operations may be used to distribute the active agent throughout those compositions employing large quantities of fillers. Such operations are conventional in the art. The tablets may for example be prepared by wet or dry granulation and optionally coated according to methods well known in normal pharmaceutical practice, in particular with an enteric coating.

The composition of the second aspect may also be adapted for parenteral administration, such as sterile solutions, suspensions or reconstitutable dry preparations, aerosols or sprays in the appropriate unit dosage form. Adequate excipients can be used, such as bulking agents, buffering agents or surfactants.

In a preferred embodiment of the second aspect, the composition comprises a therapeutically effective amount of the conjugate as defined in the first aspect of the invention. The physician will determine the dosage of the present therapeutic agents which will be most suitable and it will vary with the form of administration and the particular compound chosen, and furthermore, it will vary with the patient under treatment, the age of the patient, the type of disease or condition being treated. For instance, when the composition is administered orally, larger quantities of the active agent will be required to produce the same effect as a smaller quantity given parenterally. The compounds are useful in the same manner as comparable anti-infective agents and the dosage level is of the same order of magnitude as is generally employed with these other anti-infective agents.

The compounds and compositions of this invention may be used with other drugs to provide a combination therapy. The other drugs may form part of the same composition, or be provided as a separate composition for administration at the same time or at different time.

Throughout the description and claims the word "comprises" and variations of the word, are not intended to exclude other technical features, additives, components or steps. Furthermore, the word "comprise" encompasses the cases of "consist of" and "consists essentially of". Additional objects, advantages and features of the invention will become apparent to those skilled in the art upon examination of the description or may be learned by practice of the invention. The following examples are provided by way of illustration, and they are not intended to be limiting of the present invention.

## EXAMPLES

*Abbreviations*

RTC: resin test cleavage

LC-MS: Liquid chromatography coupled with mass spectrometry
MeCN: acetonitrile
TFA: trifluoroacetic acid
TEA: triethylamine
NMP: *N*-methlyl-2-pyrrolidone
DCM: dichloromethane
DMF: *N*,*N*-dimethylformamide
DMSO: dimethylsulfoxide
DIC: *N*,*N'*-Diisopropylcarbodiimide
DIPEA: diisopropylethylamine
TIS: triisopropylsilane
Mmt: monomethoxytrityl
HPLC: High Performance Liquid Chromatography
FA: formic acid
MS: mass spectrometry
FBS: Fetal bovine serum
ECGS: endothelial cell growth supplement
EDTA: ethylenediamine tetraacetic acid
PBS: phosphate buffer solution
Pen-Strep: penicillin-streptomycin
PFU: plaque forming unit
IC50: half-maximum inhibitory concentration
HEPES: buffer solution of 4-(2-hydroxyethyl)-1-piperazineethanesulfonic acid
FD4: 4 kDa fluorescein isothiocyanate-dextran
EGTA: Ethylene glycol-bis(2-aminoethylether)-N,N,N',N'-tetraacetic acid

*Chemicals and reagents*

Mesoporphyrin IX dihydrochloride and protoporphyrin IX were purchased from Frontier Scientific, Inc (Logan, UT, USA). HPLC-grade DMF, DCM and MeCN were purchased from Fisher (Madrid, Spain), while NMP and DIC were purchased from Sigma-Aldrich (Madrid, Spain). Fmoc-amino acids and Oxyma were purchased from Iris Biotech (Marktredwitz, Germany).

*Cells and cell culture reagents*

HBEC-5i human brain endothelia (ATCC-CRL-3245) and JEG-3 placental trophoblast (ATCC-HTB-36) cell lines, and Eagle's Minimal Essential Medium (EMEM) were purchased from ATCC (VA, USA). Dulbecco's Modified Eagle Medium: Nutrient Mixture F-12 (DMEM:F12), fetal bovine serum (FBS), penicillin-streptomycin (Pen-Strep), attachment factor protein and trypsin-EDTA were purchased from Gibco (Thermo-Fisher, MA, USA). AlamarBlue® reagent was from Invitrogen (Thermo-Fisher). Endothelial cell growth supplement from bovine neural tissue (ECGS) was from Sigma-Aldrich (Merck, Darmstadt, Germany). Penicillin and streptomycin were purchased from LGC Biotecnologia (SP, Brazil).

**Preparative Examples**

Porphyrin peptide conjugates have been prepared by conjugation of mesoporphyrin IX (also referred to herein as MP) or protoporphyrin IX (also referred to herein as PP) with peptides having as sequences SEQ ID NO1-$NH_2$, SEQ ID NO2-$NH_2$, SEQ ID NO3-$NH_2$ or SEQ ID NO4-$NH_2$ as shown in Table 1 by formation of an amide bond between a carboxyl group of MP or PP and an amine group of the N-terminus of the peptide sequence or on the amine group of the side chain of a terminal Lysine (Lys or K) forming the C-terminus of the peptide sequence.

Table 1

| Compound name | Porphyrin | Peptide sequence | Conjugation site |
|---|---|---|---|
| (I) | PP | VQQLTKRFSL-$NH_2$ (SEQ ID NO1-$NH_2$) | *N*-terminus of V |

(continued)

| Compound name | Porphyrin | Peptide sequence | Conjugation site |
|---|---|---|---|
| MP-P1 | MP | VQQLTKRFSL-$NH_2$ (SEQ ID NO1-$NH_2$) | *N*-terminus of V |
| P2-MP | MP | VQQLTKRFSLK-$NH_2$ (SEQ ID NO2-$NH_2$) | Amine group in side chain of K at C-terminus |
| P2-PP | PP | VQQLTKRFSLK-$NH_2$ (SEQ ID NO2-$NH_2$) | Amine group in side chain of K at C-terminus |
| MP-P3 | MP | SGTQEEY-$NH_2$ (SEQ ID NO3-$NH_2$) | *N*-terminus of S |
| PP-P3 | PP | SGTQEEY-$NH_2$ (SEQ ID NO3-$NH_2$) | *N*-terminus of S |
| P4-MP | MP | SGTQEEYK-$NH_2$ (SEQ ID NO4-$NH_2$) | Amine group in side chain of K at C-terminus |
| P4-PP | PP | SGTQEEYK-$NH_2$ (SEQ ID NO4-$NH_2$) | Amine group in side chain of K at C-terminus |

In a first preparation step, peptides having as sequences SEQ ID NO1-$NH_2$, SEQ ID NO2-$NH_2$, SEQ ID NO3-$NH_2$ or SEQ ID NO4-$NH_2$ were independently synthesized by Fmoc solid phase synthesis on a Liberty Blue (CEM Matthews, NC) instrument using Protide resin (0.54 mmol/g) at 0.1 mmol scale. The sequences SEQ ID NO2-$NH_2$ and SEQ ID NO4-$NH_2$ are variants of sequences SEQ ID NO1-$NH_2$ and SEQ ID NO3-$NH_2$, respectively, elongated at their C-termini with an extra Lys residue, orthogonally protected with the monomethoxytrityl (Mmt) group. Other side chain protecting groups were *tert*-butyl (for protecting Ser, Thr, Glu), trityl (for protecting Gln), Boc (for protecting Lys) and 2,2,4,6,7-pentamethyldihydrobenzofuran-5-sulfonyl (for protecting Arg). Identity of the resin-bound peptides prior to porphyrin conjugation was assessed by resin test cleavage (RTC) combined with LC-MS analysis. To do so, ca. 2 mg of peptide resin were reacted with 170 μL of TFA-TIS-$H_2O$ (95:2.5:2.5, v/v/v) for 90 minutes at room temperature. Then 1 mL of cold diethyl ether was added and the suspension was centrifuged at 12400 rpm for 8 min. The supernatant was removed to provide peptide having as sequence SEQ ID NO1-$NH_2$, SEQ ID NO2-$NH_2$, SEQ ID NO3-$NH_2$ or SEQ ID NO4-$NH_2$as a pellet. Said pellet was then analyzed by LC-MS to confirm peptide formation using 15% MeCN/0.1% TFA in water (v/v/v) (for SEQ ID NO1-$NH_2$ and SEQ ID NO2-$NH_2$) or 0.1 % TEA in water (v/v) (for SEQ ID NO3-$NH_2$ and SEQ ID NO4-$NH_2$).

In a second preparation step, the peptide having as sequence SEQ ID NO1-$NH_2$, SEQ ID NO2-$NH_2$, SEQ ID NO3-$NH_2$ or SEQ ID NO4-$NH_2$was conjugated on-resin with PP or MP according to the following procedures.

Procedure for coupling at *N*-terminus (preparation of MP-P1, (I), MP-P3 and PP-P3): Reactions were performed at 50 μM scale (1 eq in all further calculations). 4 eq of porphyrin (MP or PP), mixed with 4 eq. oxyma, were dissolved respectively in NMP:DCM:DMF (3:2:1, v/v/v, for MP) or DMF:DMSO:DCM (5:2:1, v/v/v, for PP), at a final 0.05 M concentration, thereby providing solution 1. The solution 1 was mixed with 4 eq. DIC (plus 8 eq DIPEA in the case of MP) and added to the peptide resin. After 3 hours at room temperature, the solution was dispensed and freshly prepared solution 1 was added to the resin for 1 h additional reaction. To monitor the progress of the reaction, 2 mg of peptide resin taken as an aliquot were reacted with 170 μL of TFA-TIS-$H_2O$ (95:2.5:2.5, v/v/v) for 90 min at room temperature. Product was isolated by drying under a $N_2$ stream and the residue dissolved in 20% MeCN in water (v/v) for LC-MS analysis, which was used to confirm conjugate identity, as shown in Table 2. Next, the bulk PPC-resin was likewise cleaved, the resulting solution was $N_2$ flush-evaporated, dissolved in $H_2O$/MeCN (80:20 v/v) and lyophilized.

Procedure for coupling at the amino group in side chain of K at C-terminus (preparation of P2-MP, P2-PP, P4-MP and P4-PP): The procedure is identical to the coupling at *N*-terminus, provided that the Mmt protecting group at the K side chain is removed prior to performing the conjugation step by previously treating the peptide of sequence SEQ ID NO2-$NH_2$ or SEQ ID NO4-$NH_2$ repeatedly with a 1% solution of TFA in DCM (v/v) followed by DCM washes, until no more yellow color associated to the presence of Mmt is observed. The resin was then neutralized for 30 seconds with 5% DIPEA in DCM and porphyrin coupling was performed as above.

Table 2

| Conjugate | Structure | [M+H+]+ |
|---|---|---|
| MP-P1 | 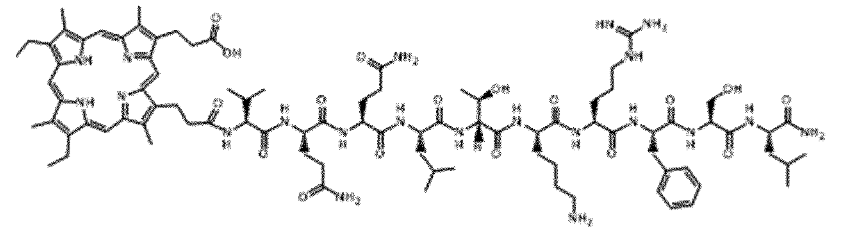 | 1767.0 |
| (I) | 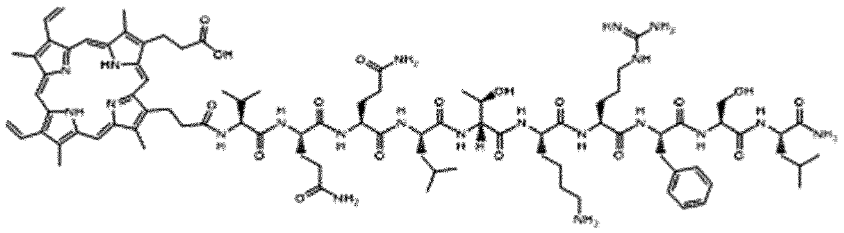 | 1762.9 |
| P2-MP | 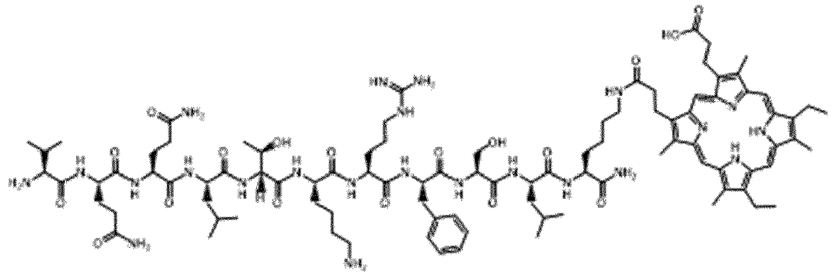 | 1896.1 |
| P2-PP | 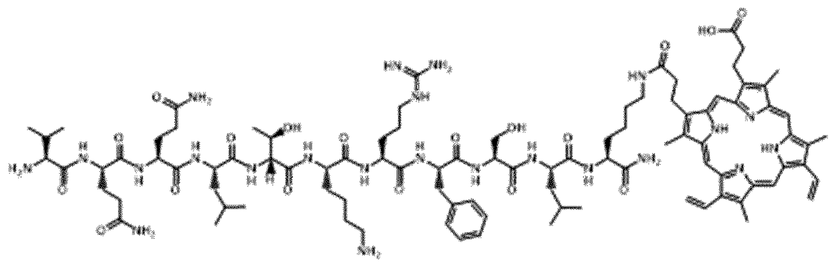 | 1892.1 |
| MP-P3 | 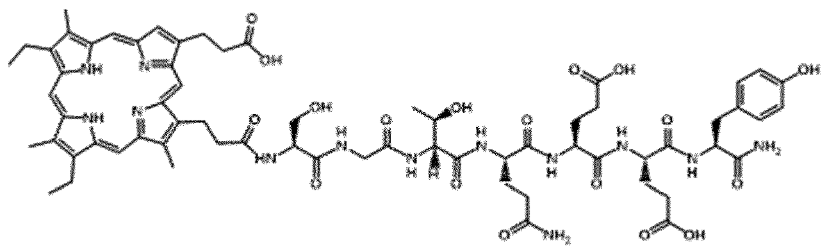 | 1360.6 |
| PP-P3 | 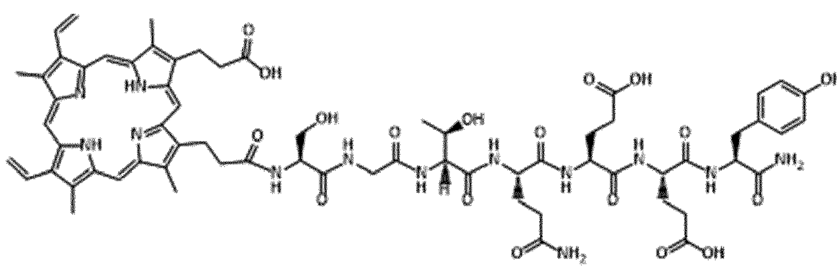 | 1356.6 |

(continued)

| Conjugate | Structure | $[M+H^+]^+$ |
|---|---|---|
| P4-MP | 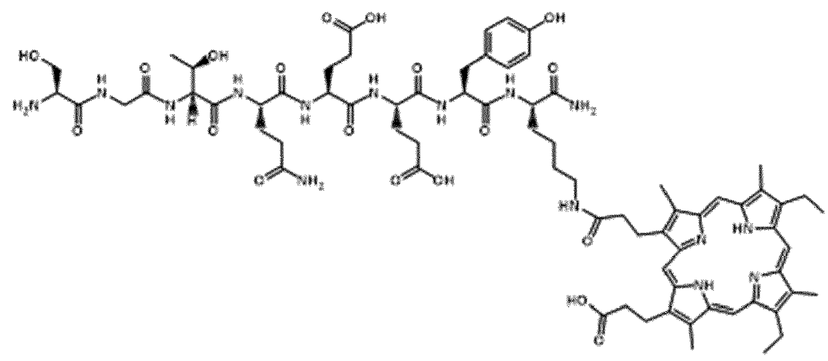 | 1488.7 |
| P4-PP | 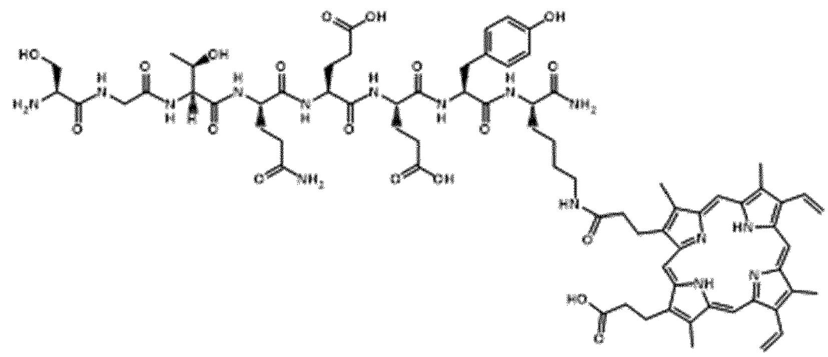 | 1484.7 |

Crude conjugate products were then purified by semi-preparative HPLC on an LC20-AP instrument (Shimadzu, Kyoto, Japan) using a Gemini® $C_{18}$ column (10 μm, 110 Å, 10 × 250 mm, Phenomenex). Each conjugate was dissolved in 22% MeCN/25 % DMF/$H_2O$ (v/v/v) and a linear 15-95% MeCN gradient in $H_2O$ (0.1 % TFA, v/v/v) over 40 min at 6 mL/min flow rate was applied. The fractions were analyzed by LC-MS and those with >90% homogeneity were collected, combined, lyophilized and stored at -20 °C.

Following this procedure, MP-P1 was prepared with 13% yield and 97.5% purity.

Following this procedure, the compound of formula (I) was prepared with 14.8% yield and 93.8% purity.

Following this procedure, P2-MP was prepared with 15.1% yield and 98.7% purity.

Following this procedure, P2-PP was prepared with 14.2% yield and 92.4% purity.

Following this procedure, MP-P3 was prepared with 28.8% yield and 94.85% purity.

Following this procedure, PP-P3 was prepared with 8% yield and 92% purity.

Following this procedure, P4-MP was prepared with 27.7% yield and 97.6% purity.

Following this procedure, P4-PP was prepared with 14.3% yield and 91.1% purity.

Procedure for LC-MS analysis of crude conjugates or peptides after RTC: Crude peptides or conjugates after RTC were dissolved in 15% or 20% MeCN in water (v/v), respectively, and analyzed on a LCMS-2010 EV instrument (Shimadzu). For the analysis, 15 μL of a ~ 1 mg/mL solution were injected on an Aeris XB-$C_{18}$ column (3.6 μm, 150 × 4.6 mm, Phenomenex) eluted with a linear 5%-95% MeCN gradient into 0.1 % FA in $H_2O$ (v/v/v) over 15 min at 1 mL/min flow rate. MS detection was set to 200-2000 *m/z*. Purified conjugates were dissolved at 1 mg/mL in 20% MeCN in water (v/v), and analyzed by LC-MS using a linear 10%-60% MeCN gradient into 0.1% FA in $H_2O$ (v/v/v) over 15 min, other parameters as above.

As will be obvious to the skilled person, conjugates MP-P1, P2-PP, P2-MP, MP-P3, PP-P3, P4-MP and P4-PP fall out of the scope of the invention and are provided as comparative examples.

General Procedure for *in vitro* Blood-Brain Barrier (BBB) translocation assay

Assessment of the efficacy of a conjugate in BBB translocation was done in a transwell set-up using HBEC-5i cells as shown on Fig. 1. HBEC-5i cells were cultured as a monolayer (3) on T-flasks in DMEM:F12 supplemented with 10% (v/v) FBS, 1% (v/v) Pen-Strep and 1% (v/v) ECGS. Cells were grown in a humidified atmosphere of 5% $CO_2$ at 37 °C (MCO-18AIC (UV), Sanyo, Japan) with the medium changed every other day. Cells were allowed to grow until

confluence in a culture T-flask, then carefully harvested with trypsin-EDTA and seeded (8000 cells/well) into tissue culture 24-well inserts (4) (transparent polyester membrane with 1.0 μm pores) (BD Falcon, USA) pre-coated with attachment factor protein solution. The medium was changed every other day for 5-8 days, afterwards cells were washed with 1 × PBS followed by DMEM:F12 medium without phenol red. Next, the conjugates were diluted in DMEM:F12 without phenol red, until reaching a final 10 μM concentration, and were added to the apical side (1) of the *in vitro* BBB model.

Conjugate translocation was determined by fluorescence intensity ($\lambda_{ex}$= 410 nm, $\lambda_{em}$ = 625 nm for MP conjugates; $\lambda_{ex}$=410 nm, $\lambda_{em}$=635 nm for PP conjugates). Conjugates of-carboxyfluorescein with peptide of sequence SEQ ID NO1-$NH_2$ (CF-P1) and SEQ ID NO3-$NH_2$ (CF-P3) were used as positive translocation controls ($\lambda_{ex}$=492 nm and $\lambda_{em}$=517 nm). After 24 h incubation, samples from the basolateral side (2) were collected and analyzed. Fluorescence was measured in a Varioskan Lux plate reader (Thermo Scientific). PPC translocation (%) was calculated as follows:

$$PPC\ translocation\ (\%) = (F_i - Fcells)/(F_PPC - F_Medium\ ) \times 100 \quad (1)$$

where $F_i$ is the fluorescence intensity of the sample collected at the basolateral side (2), $F_{cells}$ is the intrinsic fluorescence intensity of cells without incubation with a peptide-protein conjugate PPC, $F_{PPC}$ is the intensity of total PPC initially added to the transwell apical side (1), and $F_{Medium}$ is the intensity of DMEM:F12 medium without phenol red.

**General Procedure for *in vitro* determination of BBB or BPB integrity in translocation assays**

After 24 h incubation with conjugates in the set-up shown in Figure 1, an in vitro integrity assay was performed. HBEC-5i (for BBB) or JEG-3 (for BPB) cells were washed twice with PBS and once with a medium without phenol red. DMEM:F12 medium was used with HBEC-5i cells and EMEM medium was used with JEG-3 cells. Then, previously diluted 4 kDa fluorescein isothiocyanate-dextran (FD4) (Sigma-Aldrich, Spain) in the medium indicated above for each cell line and without phenol red to an absorbance of 0.1 was added to the apical side (1) and incubated for 2 h. Samples were collected at the basolateral side (2), and fluorescence intensity was measured at $\lambda_{ex}$=493 nm and $\lambda_{em}$=520 nm using a Varioskan Lux plate reader. Barrier integrity was evaluated from FD4 permeability as follows:

$$FD4\ Permeability\ (\%) = \frac{F_i - F_{cells}}{F_{FD4} - F_{Medium}} \times 100 \quad (2)$$

where $F_i$ is the fluorescence intensity of the sample at the basolateral side (2), $F_{cells}$ is the intrinsic fluorescence intensity of cells without FD4 incubation, $F_{FD4}$ is the intensity of FD4 stock initially added to the apical side (1), and $F_{Medium}$ is the intensity of the medium indicated above for each cell line and without phenol red.

**General Procedure for *in vitro* Blood-Placenta Barrier (BPB) translocation assay**

Assessment of the efficacy of a conjugate in BBB translocation was done in a transwell set-up using JEG-3 cells as shown on Fig. 1. JEG-3 cells were cultured as a monolayer on T-flasks in EMEM supplemented with 10% (v/v) FBS and 1% (v/v) Pen-Strep. Cells were grown in a humidified atmosphere of 5% $CO_2$ at 37 °C with the medium changed every other day. Cells were allowed to grow until confluence in a culture T-flask, then carefully harvested with trypsin-EDTA and seeded (3000 cells/well) onto rat-tail collagen-coated tissue culture 24-well inserts (4). A cellular monolayer (3) with low permeability was formed 5 days after the incubation on the tissue culture insert (4). BPB translocation and integrity measurement methods are identical to those for the BBB in vitro model defined above.

Table 3 below reports the obtained values for:

- BBB translocation assay for each of the prepared conjugates and control compounds, expressed as percentage of content of assayed compound in the basolateral side after the assay;
- integrity of the BBB barrier after treatment with a conjugate or a control compound, expressed as percentage of content of FD4 in the basolateral side after the assay;
- BPB translocation assay for each of the prepared conjugates and control compounds, expressed as percentage of content of assayed compound in the basolateral side after the assay;
- integrity of the BPB barrier after treatment with a conjugate or a control compound, expressed as percentage of content of FD4 in the basolateral side after the assay

Table 3

| Compound | BBB translocation (%) | BBB FD4 (%)[1] | BPB translocation (%) | BPB FD4 (%)[2] |
|---|---|---|---|---|
| (I) | 43.6 | 13.6 | 21.2 | 0.83 |
| MP | 35.7 | 17.9 | 12.1 | 1.68 |
| PP | 47.8 | 24.7 | 14.2 | 0.87 |
| MP-P1 | 30 | 19.9 | 6.9 | 1.16 |
| P2-MP | 25.5 | 14.5 | 6.9 | 1.30 |
| P2-PP | 35.1 | 13.7 | 7.9 | 0.90 |
| MP-P3 | 46.6 | 14.6 | 14.2 | 1.17 |
| PP-P3 | 43.2 | 9.6 | 18.9 | 1.79 |
| P4-MP | 45.8 | 15.6 | 13 | 1.1 |
| P4-PP | 42.8 | 10.6 | 15.6 | 1.2 |
| CF-P1 | 70.6 | 14.7 | 29.5 | 1.38 |
| CF-P3 | 70.1 | 11.1 | 28.4 | 1.56 |

[1] untreated BBB exhibits a FD4 permeability value of 10.3% of FD4 content in basolateral side; EGTA which is known to provoke disruption exhibits a value of 29.6% of FD4 content in basolateral side
[2] untreated BPB exhibits a FD4 permeability value of 1.23% of FD4 content in basolateral side; EGTA-provoked disruption exhibits a value of 9.7% of FD4 content in basolateral side

In Table 3, conjugates MP-P1, P2-PP, P2-MP, MP-P3, PP-P3, P4-MP and P4-PP fall out of the scope of the invention and are provided as comparative examples. The results of Table 3 show that all the tested compounds are suitable for crossing both the blood-brain barrier and the blood placental barrier. While protoporphyrin IX alone (PP) appears to efficiently translocate the BBB monolayer, it also appears to seriously affect its integrity, because the value obtained for FD4 permeability for PP alone is close to the value obtained with EGTA-provoked disruption. Noteworthy, the compound of formula (I) provides a good compromise between translocation capacity over the BBB and the BPB combined with preservation of the respective membrane integrity of BBB and BPB.

**Virus culture**

$ZIKV^{BR}$ was isolated from a febrile case in the state of Pernambuco, Brazil (gene bank ref. number KX197192). $ZIKV^{BR}$ was propagated in C6/36 cells using a multiplicity of infection (MOI) of 0.01. After infection, cells were cultured for 7 days. The culture medium collected from the infected cultures was centrifuged at 700 g to remove cellular debris, stored in aliquots at -80 °C and titrated by plaque assay.

**ZIKV inactivation assay**

To determine the effect of conjugates on $ZIKV^{BR}$ as prepared above, $10^5$ plaque forming units (PFU) were pre-treated with different concentrations of conjugates in the 0.01-50 μM range for 1 h at 37 °C, in the dark. After treatment, viral samples were serially diluted (10-fold) for titration by plaque assay. Samples were incubated for 1 h at 37 °C and 5% $CO_2$ with Vero cells. After infection, the medium was removed and 1 mL DMEM containing 1% (v/v) FBS, 100 U/mL penicillin, 100 μg/mL streptomycin and 1.5% carboxymethylcellulose was added to each well. The plates were incubated at 37 °C and 5% $CO_2$. After 5 days cells were fixed by 1 mL of 4% (v/v) formaldehyde in $H_2O$ for 30 min. Each plate was washed and stained with a 1% (v/v) crystal violet, 20% (v/v) ethanol solution. The number of plaques on each well was counted and corrected for well dilution to determine the pfu/mL number. The half-maximum inhibitory concentration ($IC_{50}$) was determined by non-linear regression with sigmoidal profile and variable slope using the software Graphpad Prism (version 6.0), only on treatments that achieved total inhibition.

Table 4 below reports the results of ZIKV inactivation assays reported as the $IC_{50}$ value, that is the concentration of the compound at which half of the ZIKV virus population is inactivated.

Table 4

| Compound | $IC_{50}$ (μM) |
|---|---|
| (I) | 1.08 |
| MP | >50 |
| PP | >50 |
| MP-P1 | >50 |
| P2-MP | >50 |
| P2-PP | >50 |
| MP-P3 | 25.07 |
| PP-P3 | >50 |
| P4-MP | >50 |
| P4-PP | >50 |

In Table 4, conjugates MP-P1, P2-PP, P2-MP, MP-P3, PP-P3, P4-MP and P4-PP fall out of the scope of the invention and are provided as comparative examples. While it is known in the art that protoporphyrin IX (PP) metallated with Co or Sn is an efficient anti-infective agent against ZIKV, results of Table 4 show that the non-metallated analog PP is poorly active, for having an $IC_{50}$ value higher than 50 μM. When conjugated with a peptide having as sequence SEQ ID NO1, inventors have found that the resulting conjugate, such as the compound of formula (I), exhibits a significant anti-infective activity, as reflected by the low $IC_{50}$ value. This value is sufficiently low for the compound to be active at non-cytotoxic doses, as further described below. The anti-infective activity of the compound of formula (I) against ZIKV is surprising because it is to be attributed at least to the presence of the specific peptide sequence SEQ ID NO1 in the molecular formula of the compound of formula (I). Remarkably, PP-P3 and P4-PP are poorly active against ZIKV infection. The results of Table 4 also show that the conjugation of MP with a peptide having as sequence SEQ ID NO1 does not significantly improve the activity of the conjugate over the non-conjugated compound. It is thus unexpected and surprising that the combination of PP and the peptide of sequence SEQ ID NO1-$NH_2$ in a conjugate provide such an active anti-infective agent against ZIKV.

**Cell viability assay**

HBEC-5i cells and JEG-3 cells were plated in attachment factor protein and rat-tail collagen, respectively, pre-coated 96-well flat bottom clear, black polystyrene plate (Corning, New York, USA). Afterwards cells were cultured in complete medium at 37 °C in a 5% $CO_2$ atmosphere, with medium replaced every 2 days. When the cellular monolayer was formed, cells were treated with various concentrations of conjugates in the 6.25-50 μM range, for 24 h at 37 °C in a 5% $CO_2$ atmosphere. Viability was evaluated by the CellTiter-Blue® assay (Promega, WI, USA), based on resazurin reduction into highly fluorescent resorufin by metabolically active cells. By distinguishing metabolic from non-metabolic cells, cytotoxicity can be indirectly determined. After incubation, cells were washed with PBS, pH 7.4, and 15 μL of CellTiterBlue® reagent in 100 μL of complete medium was added to the cells and incubated for 1.5 h at 37 °C in 5% $CO_2$. Fluorescence ($\lambda_{ex}$=560 nm, $\lambda_{em}$=590 nm), was measured in a Varioskan Lux plate reader. Complete medium and medium containing 0.25% Triton X-100 were used as positive and negative controls (100 and 0% viability), respectively. Cell viability (%) was determined as:

$$Cell\ viability\ (\%) = (F_treated - F_blank)/(F_(non\ treated) - F_blank\ )\ \ \times 100 \quad (3)$$

where $F_{treated}$ is the fluorescence intensity of cells treated with a conjugate, $F_{non}$ treated is the fluorescence of untreated cells and $F_{blank}$ is the fluorescence of CellTiterBlue® reagent in complete medium without cells.

According to this procedure, inventors found that none of the conjugates (I), MP-P1, P2-MP, P2-PP, MP-P3, PP-P3, P4-MP and P4-PP altered cell viability (HBEC-5i, JEG-3) at concentrations of up to 50 μM.

Serum stability

The conjugates prepared above and peptides of sequences SEQ ID NO1-$NH_2$, SEQ ID NO2-$NH_2$ and SEQ ID NO3-$NH_2$ were dissolved at 0.1 mM in HEPES buffer (10 mM, NaCl 150 mM, pH 7.8), with 1.4 % DMSO (v/v) being added for MP-P1 and P2-MP to improve solubility. Next, 600 μL of the solution comprising the conjugate or peptide were mixed (1:1 v/v) with 600 μL of human serum, to reach a final concentration of 0.05 mM. The mixture was incubated at 37 °C and, at various time points, 50 μL (in triplicate) were taken and precipitated with 200 μL cold methanol. The samples were centrifuged at 13000 rpm, 10 min, +4 °C. Afterwards, 50 μL of each supernatant were injected on an LC-20AD instrument (Shimadzu) equipped with a Luna C18 column (4.6 mm × 50 mm, 3 μm, Phenomenex, USA) and analyzed using a 0%-95% linear gradient of MeCN into 0.1% TFA in $H_2O$ over 15 min with 1 mL/min flow rate. PDA detection at 220 nm and 401 nm was used and the half-life times were calculated by peak area integration at 401 nm, the $\lambda_{max}$ of MP and PP. Controls included blank serum (25 μL each of 1:1 v/v serum + HEPES buffer, then precipitation with 200 μL cold methanol), untreated conjugate/peptide (25 μL of 0.1 mM stock in HEPES buffer + 25 μL HEPES buffer, then precipitation with 200 μL cold methanol), and a zero-time sample (200 μL cold methanol + 25 μL serum + 25 μL 0.1 mM conjugate/peptide stock), the conjugate/peptide added last to avoid any contact with protease. Controls were analyzed by LC and LC-MS as described above.

Serum stability of the prepared conjugates has been evaluated following the above procedure and half-life times of each conjugate are concealed in Table 5.

Table 5

| Compound | Serum half-life time (hours) |
|---|---|
| (I) | 22.2 |
| Peptide having SEQ ID NO1 | 0.73 |
| MP-P1 | 16.2 |
| MP-P3 | 21.1 |
| PP-P3 | 3.7 |
| P2-MP | 10.7 |
| P2-PP | 3.6 |
| P4-MP | 3 |
| P4-PP | 3.3 |

In Table 5, conjugates MP-P1, P2-PP, P2-MP, MP-P3, PP-P3, P4-MP and P4-PP fall out of the scope of the invention and are provided as comparative examples. The results of Table 5 show that the compound of the invention, i.e. the compound of formula (I), benefits from the longest half-life time in serum amongst the tested porphyrin-peptide conjugates, thereby being suitable as anti-infective drug in this aspect. In addition, these results show that conjugation at the *N*-terminus of the peptide having as sequence SEQ ID NO1 (amine group of terminal valine) provides the conjugate with higher stability in serum, when comparing the respective half-life times of the compound of formula (I) and P2-PP, wherein the protoporphyrin is conjugated at the amine group of the side chain of a terminal Lysine (Lys or K) forming the C-terminus of the peptide sequence. Also remarkably, the compound of formula (I) proves to be much more stable in serum than the peptide having as sequence SEQ ID NO1. It is believed that the porphyrin payload in the conjugate of formula (I) causes a steric shielding that occludes protease-sensitive sites in the sequence, thereby providing increase serum stability.

## CITED DOCUMENTS

1. Neris, R.L.S., Figueiredo, C.M., Higa, L.M. et al. Co-protoporphyrin IX and Sn-protoporphyrin IX inactivate Zika, Chikungunya and other arboviruses by targeting the viral envelope. Sci Rep 8, 9805 (2018).
2. Cruz-Oliveira, C.; Almeida, A. F.; Freire, J.M. et al. Mechanisms of Vesicular Stomatitis Virus Inactivation by Protoporphyrin IX, Zinc-Protoporphyrin IX, and Mesoporphyrin IX Antimicrobial Agents and Chemotherapy, 61(6), 1 (2017)
3. Yu, Y., Deng, YQ., Zou, P. et al. A peptide-based viral inactivator inhibits Zika virus infection in pregnant mice

and fetuses. Nat Commun 8, 15672 (2017).
4. Schroeder, B.; Demirel, P.; Fischer, C.; Masri et al. Nanoparticular Inhibitors of Flavivirus Proteases from Zika, West Niel and Dengue Virus Are Cell-Permeable Antivirals ACS Med. Chem. Lett 12, 1955 (2021).

## Claims

1. A conjugate of protoporphyrin IX with the peptide having the sequence Val-Gln-Gln-Leu-Thr-Lys-Arg-Phe-Ser-Leu-$NH_2$ (SEQ ID NO1-$NH_2$) or a pharmaceutically acceptable salt or a pharmaceutically acceptable co-crystal thereof for use in the prevention or treatment of ZIKA virus infection.

2. A conjugate as defined in claim 1 wherein protoporphyrin IX is linked to the terminal valine of the peptide having SEQ ID NO1-$NH_2$.

3. A conjugate as defined in claims 1 to 2 wherein said protoporphyrin IX is linked to the peptide through one of its carboxylic acid groups.

4. A conjugate as defined in claims 1 to 3 that is a compound of formula (I)

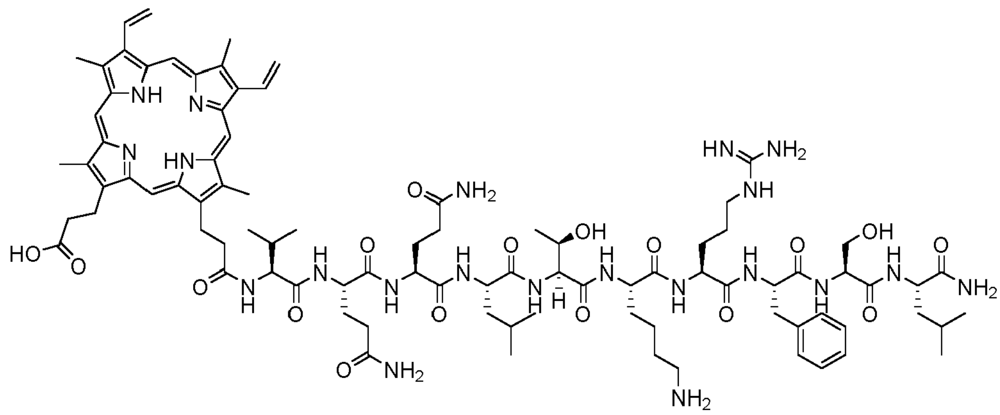

(I)

5. A composition comprising a conjugate as defined in any one of claims 1 to 5 and a pharmaceutically acceptable diluent or carrier for use in the prevention or treatment of ZIKA virus infection.

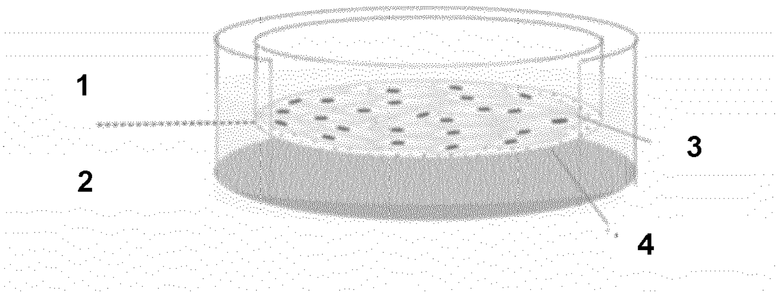


FIG. 1

Europäisches Patentamt
European Patent Office
Office européen des brevets

# EUROPEAN SEARCH REPORT

Application Number
EP 22 38 2150

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | NERIS ROMULO L. S. ET AL: "Co-protoporphyrin IX and Sn-protoporphyrin IX inactivate Zika, Chikungunya and other arboviruses by targeting the viral envelope", SCIENTIFIC REPORTS, vol. 8, no. 1, 1 December 2018 (2018-12-01), page 9805, XP055943938, DOI: 10.1038/s41598-018-27855-7 Retrieved from the Internet: URL:https://www.nature.com/articles/s41598-018-27855-7.pdf> [retrieved on 2022-07-18] * abstract * * table 1 * * figures 1-6 * * page 9, paragraph 4 - page 10, paragraph 1 * ----- | 1-5 | INV. A61K47/64 A61P31/14 |
| A | MENDONÇA DIOGO A. ET AL: "Penetrating the Blood-Brain Barrier with New Peptide-Porphyrin Conjugates Having anti-HIV Activity", BIOCONJUGATE CHEMISTRY, vol. 32, no. 6, 16 June 2021 (2021-06-16), pages 1067-1077, XP055943708, US ISSN: 1043-1802, DOI: 10.1021/acs.bioconjchem.1c00123 Retrieved from the Internet: URL:https://pubs.acs.org/doi/pdf/10.1021/acs.bioconjchem.1c00123> [retrieved on 2022-07-18] * abstract * * table 1 * * scheme 1 * * page 1070, column 2, paragraph 7 - page 1071, column 1, paragraph 1 * ----- | 1-5 | TECHNICAL FIELDS SEARCHED (IPC) A61K A61P |

The present search report has been drawn up for all claims

1

| Place of search | Date of completion of the search | Examiner |
|---|---|---|
| The Hague | 19 July 2022 | Monami, Amélie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- 1983 IUPAC-IUB Commission of Biochemical Nomenclature. *Eur. J. Biochem.,* 1984, vol. 138, 937
- **NERIS, R.L.S. ; FIGUEIREDO, C.M. ; HIGA, L.M. et al.** Co-protoporphyrin IX and Sn-protoporphyrin IX inactivate Zika, Chikungunya and other arboviruses by targeting the viral envelope. *Sci Rep,* 2018, vol. 8, 9805
- **CRUZ-OLIVEIRA, C. ; ALMEIDA, A. F. ; FREIRE, J.M. et al.** Mechanisms of Vesicular Stomatitis Virus Inactivation by Protoporphyrin IX, Zinc-Protoporphyrin IX, and Mesoporphyrin IX. *Antimicrobial Agents and Chemotherapy,* 2017, vol. 61 (6), 1
- **YU, Y. ; DENG, YQ. ; ZOU, P. et al.** A peptide-based viral inactivator inhibits Zika virus infection in pregnant mice and fetuses. *Nat Commun,* 2017, vol. 8, 15672
- **SCHROEDER, B. ; DEMIREL, P. ; FISCHER, C. ; MASRI et al.** Nanoparticular Inhibitors of Flavivirus Proteases from Zika, West Niel and Dengue Virus Are Cell-Permeable Antivirals. *ACS Med. Chem. Lett,* 2021, vol. 12, 1955